# EUROPEAN PATENT APPLICATION

(11) **EP 4 166 093 A1**
(43) Date of publication of application: **19.04.2023**
(21) Application number: 21820882.5
(22) Date of filing: 11.06.2021
(51) Int. Cl.: A61B 10/02, A61B 18/00, A61B 17/34

(54) **VIBRATING NEEDLE DEVICE AND NEEDLE INSERTION METHOD**

(30) Priority: 11.06.2020 JP 2020101453
(71) Applicant: Tohoku University, Sendai-shi, Miyagi 980-8577 (JP)
(72) Inventor: KIKUCHI, Kenji, Sendai-shi, Miyagi 980-8577 (JP); SUGIYAMA, Kojiro, Sendai-shi, Miyagi 980-8577 (JP); ISHIKAWA, Takuji, Sendai-shi, Miyagi 980-8577 (JP); TAKASE, Kei, Sendai-shi, Miyagi 980-8577 (JP)
(74) Representative: dompatent von Kreisler Selting Werner - Partnerschaft von Patent- und Rechtsanwälten mbB
(86) International application number: PCT/JP2021/022252
(87) International publication number: WO 2021/251478

(57) **Abstract**

A vibrating needle device (1) of the invention includes: a needle (10) including a main body portion (11) extending in a direction of a first axis (C1) and having a groove formed in an outer circumferential surface over an entire circumference in a circumferential direction around the first axis (C1) and a tip portion (12) provided at one end of the main body portion (11) and tapered in a direction away from the main body portion (11) in the direction of the first axis (C1); and a vibrating unit (20) configured to vibrate the needle (10) along the direction of the first axis (C1) and a direction of a second axis (C2) that intersects with the direction of the first axis (C1).

## Description

### Technical Field

The present invention relates to a vibrating needle device and a needle insertion method. The present application claims priority of Japanese Patent Application No. 2020-101453 filed on June 11, 2020, and contents thereof are incorporated by reference.

### Background Art

In a medical site, percutaneous needle puncture may be used at the time of medical care.

For example, PTL 1 below discloses a puncture unit capable of reducing pain when a needle punctures skin.

### Citation list

### Patent Literature

PTL 1: JP5835944B

### Summary of Invention

### Technical Problem

Incidentally, there is a medical care method in which a needle is inserted from skin into a tumor in a body to collect, cauterize, and freeze the tumor. The medical care method by needle puncture can reduce burden on a patient as compared with an abdominal operation. However, in the related art, since an organ, which is an object of needle puncture, is not completely fixed in the body and moves together with movement of the needle, it is fairly difficult to accurately puncture the object such as the organ with the needle.

An object of the invention is to provide a vibrating needle device and a needle insertion method that can easily perform puncture with a needle.

### Solution to Problem

In order to solve the above problem, a vibrating needle device according to the invention includes: a needle including a main body portion extending in a direction of a first axis and having a groove formed in an outer circumferential surface over an entire circumference in a circumferential direction around the first axis and a tip portion provided at one end of the main body portion and tapered in a direction away from the main body portion in the direction of the first axis; and a vibrating unit configured to vibrate the needle along the direction of the first axis and a direction of a second axis that intersects with the direction of the first axis.

In the above configuration, a side surface of the groove on a base end portion side of the needle is inclined inward in a radial direction orthogonal to the first axis from the base end portion side toward a tip portion side.

In order to solve the above problem, a vibrating needle device according to the invention includes: a needle including a main body portion extending in a direction of a first axis and a tip portion provided at one end of the main body portion and tapered in a direction away from the main body portion in the direction of the first axis; and a vibrating unit configured to vibrate the needle at a low frequency of 5 Hz or more and 15 Hz or less along at least one of the direction of the first axis and a direction of a second axis that intersects with the direction of the first axis.

In the above configuration, the vibrating unit is configured to vibrate the needle to have a saw-tooth shape or/and inverse saw-tooth shape vibration waveform in at least one of the direction of the first axis and the direction of the second axis.

In the above configuration, the vibrating unit is configured to vibrate the needle to have a square wave shape vibration waveform in at least one of the direction of the first axis and the direction of the second axis.

In the above configuration, a frequency at which the vibrating unit vibrates the needle is set to resonate with a natural frequency of the needle.

In the above configuration, the second axis is orthogonal to the first axis.

In order to solve the above problem, an insertion method according to the invention is a needle insertion method using any of the above vibrating needle devices. The needle insertion method includes: a vibrating step of vibrating the needle in the direction of the first axis and the direction of the second axis; and an insertion step of inserting the needle into an object in a state of vibrating the needle.

In order to solve the above problem, an insertion method according to the invention is a needle insertion method using the above vibrating needle device. The needle insertion method includes: a vibrating step of vibrating the needle at a low frequency of 5 Hz or more and 15 Hz or less in at least one of the direction of the first axis and the direction of the second axis; and an insertion step of inserting the needle into an object in a state of vibrating the needle.

### Advantageous Effects of Invention

Therefore, the invention provides the vibrating needle device and the needle insertion method that can easily puncture an object with a needle.

### Brief Description of Drawings

[FIG. 1] FIG. 1 is a schematic diagram of a vibrating needle device according to an embodiment.
[FIG. 2] FIG. 2 is a side view of a needle according to the embodiment.
[FIG. 3] FIG. 3 is an enlarged view of a portion III in FIG. 2.
[FIG. 4] FIG. 4 is a side view of a needle according to a modification.
[FIG. 5] FIG. 5 is an enlarged view of a portion V in FIG. 4.
[FIG. 6] Fig. 6 is a side view of a needle having no groove.
[FIG. 7] Fig. 7 is a graph showing changes in puncture force.
[FIG. 8] FIG. 8 is a graph comparing a primary peak of puncture force in each vibration.
[FIG. 9] FIG. 9 is a graph comparing a secondary peak of puncture force in each vibration.
[FIG. 10] FIG. 10 is a graph comparing stable regions of puncture force in each vibration.
[FIG. 11] FIG. 11 is a graph comparing stable regions of puncture force in each needle and each vibration.
[FIG. 12] FIG. 12 is a graph showing a correlation between the secondary peak of the puncture force and a puncture velocity in each vibration.
[FIG. 13] FIG. 13 is a graph showing a correlation between total puncture force and a puncture velocity in each vibration.
[FIG. 14] FIG. 14 is a graph showing a correlation between the secondary peak of the puncture force and a puncture velocity in vibration in two directions.
[FIG. 15] FIG. 15 is a graph showing a correlation between total puncture force and a puncture velocity in vibration in two directions.
[FIG. 16] FIG. 16 is a graph showing a correlation between puncture force and needle displacement in no vibration and in vibration in two directions.
[FIG. 17] FIG. 17 is a box plot diagram comparing a maximum value of puncture force in no vibration with a maximum value of puncture force in square wave vibration.
[FIG. 18] FIG. 18 is a graph showing a correlation between a maximum value of puncture force and a puncture velocity in no vibration and in each vibration.
[FIG. 19] FIG. 19 is a graph showing a correlation between total puncture force and a puncture velocity in no vibration and in each vibration.
[FIG. 20] FIG. 20 is a diagram showing displacement of a second kidney model caused by instantaneous single puncture for each puncture acceleration.
[FIG. 21] FIG. 21 is a graph showing a correlation between displacement of a puncture object and a puncture acceleration in an axial direction and a correlation between a puncture depth and the puncture acceleration in the axial direction in the second kidney model.
[FIG. 22] FIG. 22 is a diagram showing surface displacement of a pig kidney caused by instantaneous single puncture for each puncture acceleration.
[FIG. 23] FIG. 23 is a box plot diagram showing a correlation between surface displacement of a puncture object and a puncture acceleration in the axial direction in a pig kidney.

### Description of Embodiments

Hereinafter, an embodiment of a vibrating needle device 1 according to the invention will be described with reference to the drawings.

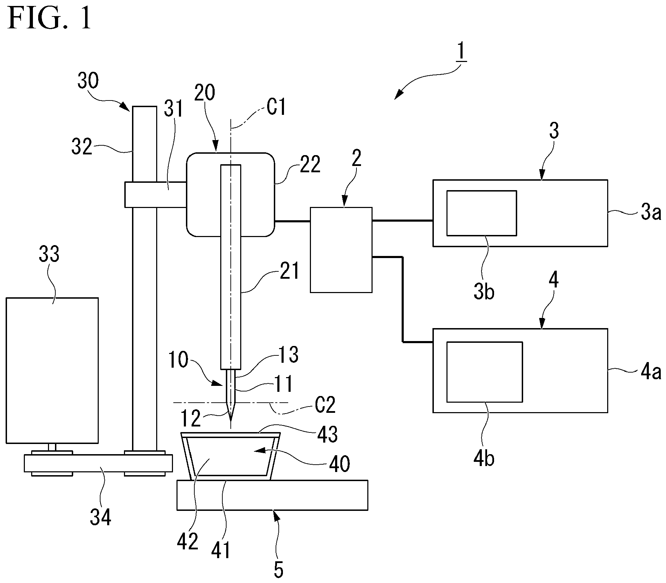

FIG. 1 is a schematic diagram of the vibrating needle device 1.

As shown in FIG. 1, the vibrating needle device 1 includes a needle 10, a vibrating unit 20, a control unit 2, a vibration waveform generation unit 3, an observation unit 4, and a moving device 30. The vibrating needle device 1 is used as, for example, a medical instrument. The vibrating needle device 1 is used for puncturing (corresponding to "inserting" in the claims) an organ of a patient, which is an object with the needle 10, for example, at the time of medical care. In the present embodiment, a human organ such as a kidney whose tissue is covered with a membrane will be described as an example of an organ which is an object.

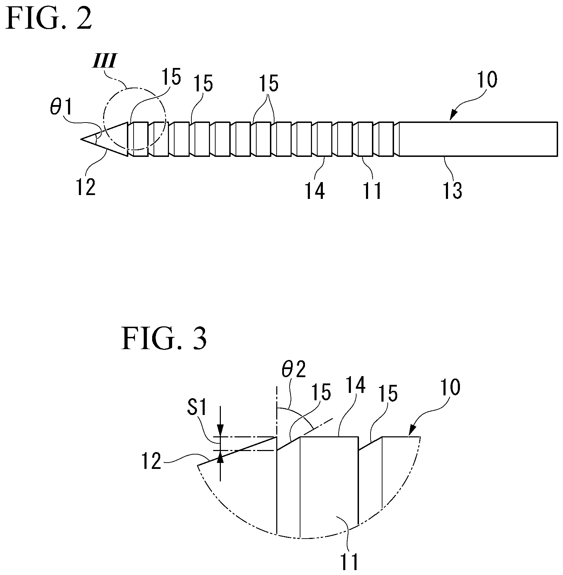

FIG. 2 is a side view of the needle 10.

FIG. 3 is an enlarged view of a portion III in FIG. 2.

As shown in FIGS. 2 and 3, the needle 10 is formed of a metal material. The needle 10 includes a cylindrical main body portion 11 extending in a direction of a first axis C1 (hereinafter, simply referred to as an "axial direction"), a tip portion 12 provided at one end of the main body portion 11 in the axial direction, and a base end portion 13 provided at the other end of the main body portion 11 in the axial direction.

In the following description, a direction orthogonal to the "axial direction" is referred to as a "radial direction", and a direction around the "axial direction" is referred to as a "circumferential direction".

A length of the main body portion 11 in the axial direction is, for example, 70 mm. A maximum outer diameter of the main body portion 11 is, for example, 5 mm.

The tip portion 12 is integrally formed with the main body portion 11. The tip portion 12 is formed in a conical shape tapered in a direction away from the main body portion 11 in the axial direction. An apex angle θ1 of the tip portion 12 is, for example, 40 degrees.

The base end portion 13 is integrally formed with the main body portion 11. The base end portion 13 is formed in a cylindrical shape having the same diameter as the maximum outer diameter of the main body portion 11.

Grooves 15 are formed in an outer circumferential surface 14 of the main body portion 11. A plurality of (14 in the present embodiment) grooves 15 are formed at intervals of, for example, 3.0 mm in the axial direction from the tip portion 12 toward the base end portion 13 in the main body portion 11. The groove 15 is formed over an entire circumference of the main body portion 11 in the circumferential direction. A maximum depth S1 of the groove 15 is, for example, 0.5 mm. The groove 15 located closest to the base end portion 13 among the plurality of grooves 15 is formed at a position of, for example, 39 mm in the axial direction from the tip portion 12 toward the base end portion 13 in the main body portion 11. A side surface of the groove 15 on the tip portion 12 side extends along the radial direction. A side surface of the groove 15 on the base end portion 13 side is formed in a tapered shape inclined inward in the radial direction from the base end portion 13 toward the tip portion 12. The side surface of the groove 15 on the base end portion 13 side intersects with the side surface of the groove 15 on the tip portion 12 side at, for example, 60 degrees (angle θ2) in a cross-sectional view along the axial direction. The shape of the groove is V-shaped in a cross-sectional view along the axial direction.

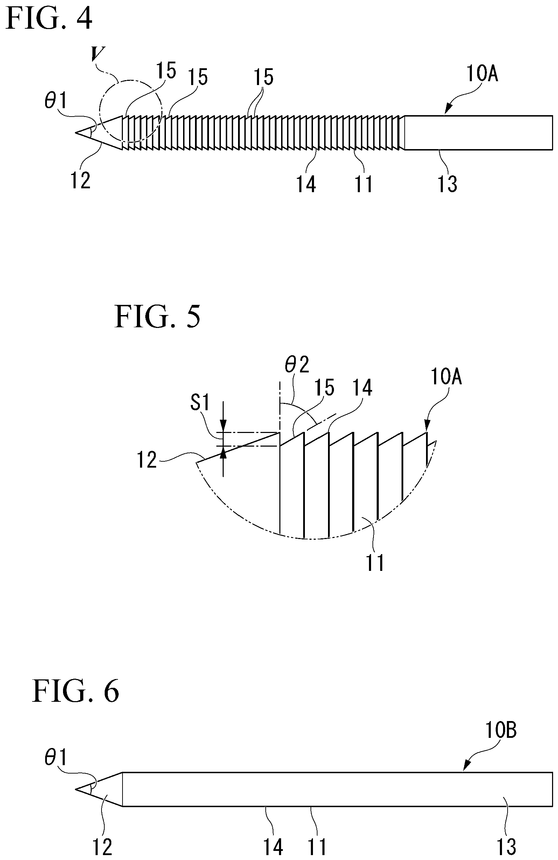

FIG. 4 is a side view of a needle 10A.

FIG. 5 is an enlarged view of a portion V in FIG. 4.

The needle of the invention is not limited to the needle 10, and may be the needle 10A according to a modification of the embodiment. As shown in FIGS. 4 and 5, a plurality of (for example, 46) grooves 15 of the needle 10A may be formed at intervals of, for example, 0.9 mm in the axial direction from the tip portion 12 toward the base end portion 13. The groove 15 located closest to the base end portion 13 among the plurality of grooves 15 may be formed at a position of, for example, 40.5 mm in the axial direction from the tip portion 12 toward the base end portion 13 in the main body portion 11.

As shown in FIG. 1, the vibrating unit 20 vibrates the needle 10 along the axial direction and a direction of a second axis C2 orthogonal to the axial direction. The direction of the second axis C2 is coincident with the radial direction. A frequency at which the vibrating unit 20 vibrates the needle 10 is set to, for example, several tens of Hz corresponding to a natural frequency of the needle 10. Accordingly, the frequency at which the vibrating unit 20 vibrates the needle 10 resonates with the natural frequency of the needle 10. In addition, for example, when an ultrasonic apparatus using ultrasonic waves vibrating at a frequency of 20 kHz or more is used at the same time, it is possible to prevent the vibrating needle device 1 and the ultrasonic apparatus from interfering with each other. The vibrating unit 20 vibrates the needle 10 to have any vibration waveform in the axial direction and the radial direction. The vibrating unit 20 vibrates the needle 10 to have a square wave shape vibration waveform in both the axial direction and the radial direction, for example. The vibrating unit 20 includes a support unit 21 and a piezoelectric actuator 22.

The support unit 21 is a cylindrical member. The base end portion 13 of the needle 10 is fixed to one end of the support unit 21. The support unit 21 is disposed coaxially with the needle 10. The other end of the support unit 21 is attached to the piezoelectric actuator 22.

The piezoelectric actuator 22 vibrates the needle 10 in the axial direction and the radial direction together with the support unit 21. The piezoelectric actuator 22 generates vibration in accordance with a voltage input from the control unit 2 to be described later. A maximum amplitude of the piezoelectric actuator 22 is, for example, 100 µm.

The control unit 2 is electrically connected to the piezoelectric actuator 22. The control unit 2 applies a voltage to the piezoelectric actuator 22 to vibrate the piezoelectric actuator 22.

The vibration waveform generation unit 3 is electrically connected to the control unit 2. The vibration waveform generation unit 3 inputs a signal to the control unit 2. The signal from the vibration waveform generation unit 3 includes information on a vibration waveform of the voltage applied from the control unit 2 to the piezoelectric actuator 22. The vibration waveform generation unit 3 is, for example, a function generator 3a. The function generator 3a includes an input monitor 3b that displays a vibration waveform included in the signal input to the control unit 2.

The observation unit 4 is electrically connected to the control unit 2. A signal including information on the vibration waveform of the voltage output from the control unit 2 to the piezoelectric actuator 22 is output from the control unit 2 to the observation unit 4. The observation unit 4 is, for example, an oscilloscope 4a. The oscilloscope 4a includes an output monitor 4b that displays the vibration waveform of the voltage output from the control unit 2 to the piezoelectric actuator 22.

The moving device 30 moves the needle 10 in the axial direction and punctures an object with the needle 10. The moving device 30 includes an attachment unit 31, a ball screw 32, and a motor 33. The piezoelectric actuator 22 is attached to the attachment unit 31. The ball screw 32 extends in the axial direction. The attachment unit 31 is attached to the ball screw 32 so as to be movable in the axial direction by rotation of the ball screw 32. The motor 33 is, for example, a DC motor. The motor 33 is a drive source of the ball screw 32. A rotation speed of the motor 33 is, for example, 1400 rpm. The motor 33 is connected to the ball screw 32 by a gear head 34. The gear head 34 is a speed reducer. The gear head 34 transmits driving force of the motor 33 to the ball screw 32 while reducing the speed. The gear head 34 can change a velocity of the needle 10 attached to the attachment unit 31 in a puncture direction (hereinafter, simply referred to as "puncture velocity") by changing a gear ratio. The gear ratio of the gear head 34 is, for example, 150, 75, or 30. The puncture velocity of the needle 10 corresponds to the gear ratio 150, 75, or 30 of the gear head 34, and is, for example, 311 pm/s, 622 pm/s, or 1556 pm/s, respectively.

### (Puncture Method of Needle)

Hereinafter, a puncture method of the needle 10 using the vibrating needle device 1 will be described.

The puncture method of the needle 10 includes a vibrating step, a moving step, and a puncture step.

### (Vibrating Step)

The vibrating step is a step of vibrating the needle 10 in the axial direction and the radial direction.

In the vibrating step, a signal is output from the function generator 3a to the control unit 2. The signal from the function generator 3a includes a first signal corresponding to vibration of the needle 10 in the axial direction and a second signal corresponding to vibration of the needle 10 in the radial direction. The control unit 2 applies a voltage based on the first signal and the second signal to the piezoelectric actuator 22. The piezoelectric actuator 22 vibrates the needle 10 in the axial direction based on the first signal. The piezoelectric actuator 22 vibrates the needle 10 in the radial direction based on the second signal.

### (Moving Step)

The moving step is a step of moving the needle 10 in the axial direction until the tip portion 12 of the needle 10 comes into contact with a membrane of an organ in a state of vibrating the needle 10.

In the moving step, the motor 33 is driven. The driving force of the motor 33 is transmitted to the ball screw 32 via the gear head 34. Accordingly, the ball screw 32 rotates. The attachment unit 31 moves toward the organ along the axial direction by the rotation of the ball screw 32. Therefore, the needle 10, in a vibrated state, moves along the axial direction together with the attachment unit 31 until the needle 10 comes into contact with the membrane of the organ.

### (Puncture Step)

The puncture step is a step of puncturing the organ in a state of vibrating the needle 10.

In the puncture step, the organ is punctured with the needle 10 in the state of vibrating the needle 10. The tip portion 12 presses the membrane while repeating a contact state and a non-contact state with the membrane. Thereafter, the tip portion 12 breaks through the membrane and passes through the membrane. The needle 10 punctures tissue inside the organ while pressing the membrane in contact with the needle 10. Thereafter, the membrane is restored by elastic force. The tissue enters the groove 15 of the needle 10. The tissue is restored by entering the groove 15 by the elastic force. Thereafter, the needle 10 punctures the inside of the tissue. As described above, the needle 10 punctures the organ.

### (Experimental Results relating to Vibrating Needle Device)

Hereinafter, results of experiments relating to the vibrating needle device 1 will be described.

FIG. 6 is a side view of a needle 10B having no groove 15.

As shown in FIG. 6, the needle 10B is different from the needle 10 and the needle 10A in that no groove is provided on the outer circumferential surface 14 of the main body portion 11. Hereinafter, Experiment 1 to Experiment 5 relating to the vibrating needle device 1 were performed using the needles 10, 10A, and 10B.

As shown in FIG. 1, a kidney model 40 was prepared as an object to be punctured by the needles 10, 10A, and 10B. The kidney model 40 includes an agar gel 42 and a silicone rubber 43. The kidney model 40 corresponds to the organ. The agar gel 42 fills a weighing dish 41 in a solidified state. A concentration of the agar gel 42 was 2.0%. The agar gel 42 corresponds to the tissue inside the organ. The silicone rubber 43 covers the agar gel 42. The silicone rubber 43 is fixed to the agar gel 42 by, for example, a clip (not shown). The silicone rubber 43 corresponds to the membrane of the organ.

The vibrating needle device 1 was installed such that the axial direction of the needles 10, 10A, and 10B was coincident with a gravity vertical direction. The kidney model 40 was disposed at a position separated downward from the tip portion 12 of each of the needles 10, 10A, and 10B. The silicone rubber 43 of the kidney model 40 is disposed on the tip portion 12 side. The kidney model 40 was disposed on an electronic balance 5. Accordingly, puncture force of the needles 10, 10A, and 10B, which puncture the kidney model 40, can be measured.

### (Experiment 1)

Experiment 1 was performed to confirm relation between the created kidney model 40 and the puncture force. Specifically, Experiment 1 was performed a plurality of times under the following conditions. The kidney model 40 was punctured without vibrating the needle 10B. A puncture velocity of the needle 10B is 311 um/s.

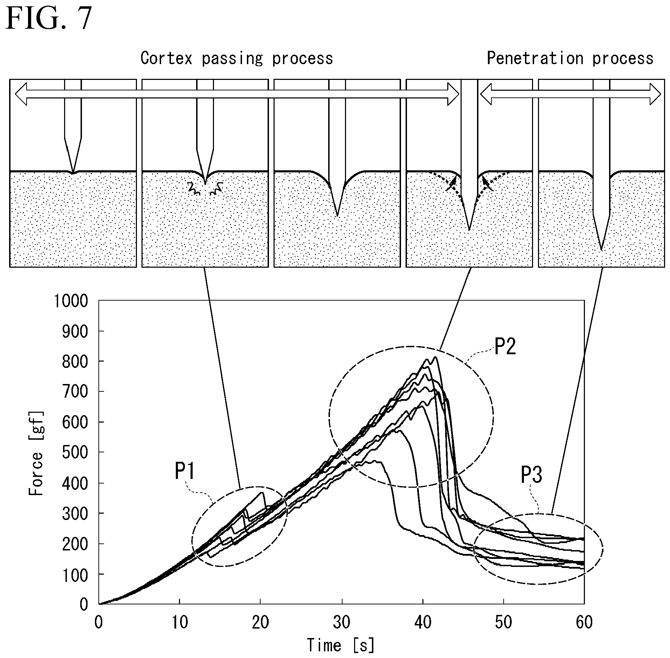

FIG. 7 is a graph showing changes in puncture force. In FIG. 7, a vertical axis represents the puncture force [gf], and a horizontal axis represents time [s] during which the needle 10B punctures the kidney model 40. FIG. 7 shows results of a plurality of trials.

As can be seen from the graph of FIG. 7, the puncture force increases over time and reaches a primary peak P1. The primary peak P1 occurs in a range of 10 s to 25 s. The puncture force at the primary peak P1 is about 100 gf to 400 gf. The puncture force decreases after passing through the primary peak P1.

Thereafter, the puncture force increases again over time, and reaches a secondary peak P2. The puncture force has a maximum value at the secondary peak P2. The puncture force decreases again after passing through the secondary peak P2. The secondary peak P2 occurs in a range of 30 s to 45 s. The puncture force at the secondary peak P2 is about 400 gf to 900 gf.

Thereafter, the puncture force reaches a stable region P3. A change rate of the puncture force in the stable region P3 with respect to time is smaller than that until the puncture force reaches the secondary peak P2. The stable region P3 occurs after 45 s. The puncture force in the stable region P3 is about 100 gf to 300 gf.

### (Experiment 2)

Experiment 2 was performed to confirm relation between a type of vibration and the puncture force.

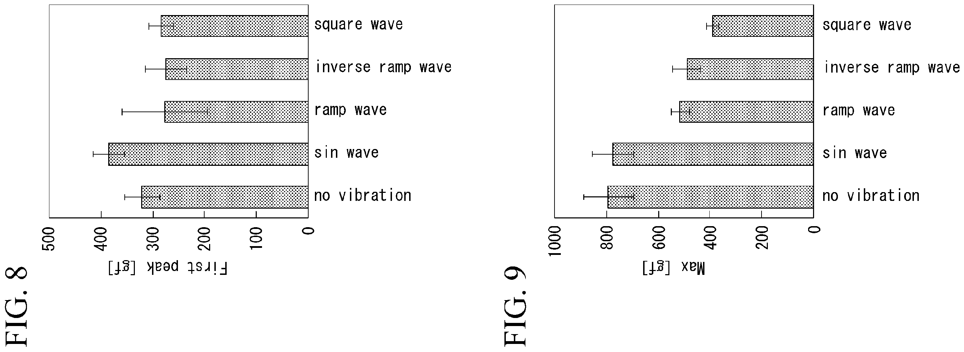

FIG. 8 is a graph showing a result of Experiment 2, which is a graph comparing the primary peak P1 of the puncture force in each vibration.

FIG. 9 is a graph showing a result of Experiment 2, which is a graph comparing the secondary peak P2 of the puncture force in each vibration.

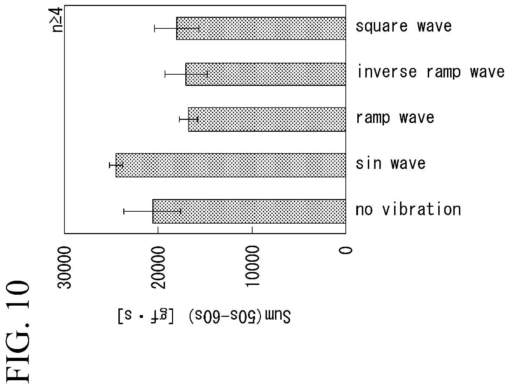

FIG. 10 is a graph showing a result of Experiment 2, which is a graph comparing the stable region P3 of the puncture force in each vibration.

Experiment 2 is different from Experiment 1 in that the needle 10B is vibrated in the axial direction. Changes in the puncture force were measured in a case where the puncture was performed while vibrating the needle 10B in a sine wave shape (sin wave), in a case where the puncture was performed while vibrating the needle 10B in a saw-tooth shape (ramp wave), in a case where the puncture was performed while vibrating the needle 10B in an inverse saw-tooth shape (inverse ramp wave), and in a case where the puncture was performed while vibrating the needle 10B in a square wave shape (square wave).

The sine wave shape vibration has a frequency of 10 Hz and an amplitude of 100 um. The saw-tooth shape vibration has the frequency of 10 Hz and an amplitude of 89.6 um. The inverse saw-tooth shape vibration has the frequency of 10 Hz and an amplitude of 89.6 um. The square wave shape vibration has the frequency of 10 Hz and an amplitude of 100 um.

The primary peak P1, the secondary peak P2, and the stable region P3 of the puncture force in each vibration were compared in a case where the puncture was performed without vibrating the needle 10B (no vibration).

In FIGS. 8 to 10, the primary peak P1, the secondary peak P2, and the stable region P3 were compared in the case where the puncture was performed without vibrating the needle 10B and in a case where the puncture was performed while vibrating the needle 10B in the axial direction. In the stable region P3 of FIG. 10, integrated values from 50 s to 60 s are compared.

In FIGS. 8 to 10, vertical axes represent values [gf] of the primary peak P1, the secondary peak P2, and the stable region P3, respectively, and horizontal axes represent experimental conditions (vibration state).

As can be seen from the graphs of FIGS. 8 to 10, the primary peak P1, the secondary peak P2, and the stable region P3 in the case where the puncture was performed while vibrating the needle 10B in the saw-tooth shape, the inverse saw-tooth shape, and the square wave shape are smaller than the primary peak P1, the secondary peak P2, and the stable region P3 in the case where the puncture was performed without vibrating the needle 10B.

The primary peak P1 and the stable region P3 in the case where the puncture was performed while vibrating the needle 10B in the sine wave shape are larger than the primary peak P1 and the stable region P3 in the case where the puncture was performed without vibrating the needle 10B. The secondary peak P2 in the case where the puncture was performed while vibrating the needle 10B in the sine wave shape was slightly smaller than the secondary peak P2 in the case where the puncture was performed without vibrating the needle 10B.

As described above, it can be said that the case where the puncture is performed while vibrating the needle 10B in the saw-tooth shape, the inverse saw-tooth shape, and the square wave shape has an effect of reducing the puncture force (hereinafter, simply referred to as a "puncture force reduction effect obtained by vibration") than the case where the puncture is performed without vibrating the needle 10B and the case where the puncture is performed while vibrating the needle 10B in the sine wave shape.

### (Experiment 3)

Experiment 3 was performed to confirm relation between the groove 15, a type of vibration and the puncture force.

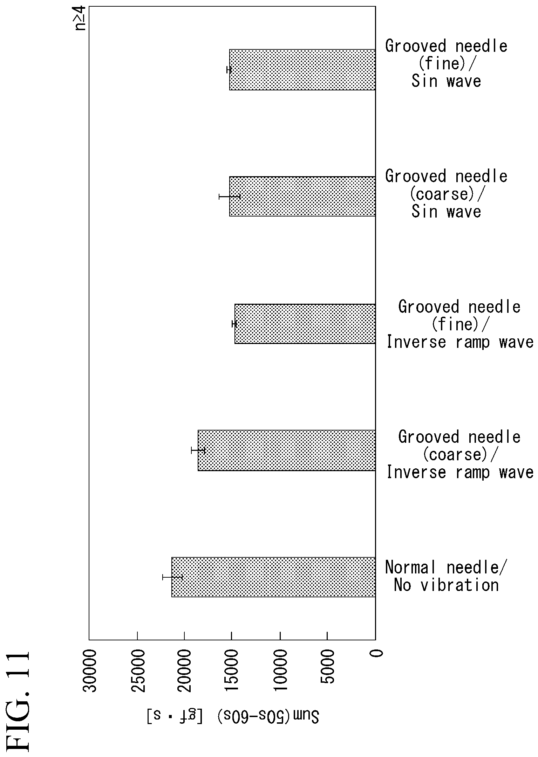

FIG. 11 is a graph showing a result of Experiment 3, which is a graph comparing the stable region P3 of the puncture force in each needle 10, 10A, and 10B, and each vibration.

Experiment 3 is different from Experiment 1 in that the kidney model 40 was punctured while vibrating the needle 10 and the needle 10A in the axial direction.

A plurality of grooves 15 are formed in the outer circumferential surface 14 of the needle 10 at intervals of 3.0 mm. A plurality of grooves 15 are formed in the outer circumferential surface 14 of the needle 10A at intervals of 0.9 mm.

Changes in the puncture force were measured in a case where the puncture was performed while vibrating the needle 10 in the inverse saw-tooth shape (grooved needle (coarse)/inverse ramp wave), in a case where the puncture was performed while vibrating the needle 10 in the sine wave shape (grooved needle (coarse)/sin wave), in a case where the puncture was performed while vibrating the needle 10A in the inverse saw-tooth shape (grooved needle (fine)/inverse ramp wave), and in a case where the puncture was performed while vibrating the needle 10A in the sine wave shape (grooved needle (fine)/sin wave).

The sine wave shape vibration has the frequency of 10 Hz and the amplitude of 100 um. The inverse saw-tooth shape vibration is 10 Hz and has an amplitude of 89.6 µm.

The stable region P3 of the puncture force in each vibration were compared in the case where the puncture was performed without vibrating the needle 10B (normale needle/no vibration).

In FIG. 11, the integrated values from 50 s to 60 s in the stable region P3 are compared in the case where the puncture was performed without vibrating the needle 10B, and in the case where the puncture was performed while vibrating the needles 10 and 10A in the axial direction.

In FIG. 11, a vertical axis represents values [gf] of the stable region P3, and a horizontal axis represents experimental conditions (groove/vibration state).

As can be seen from the graph of FIG. 11, the puncture force in the case where the puncture was performed while vibrating the needles 10 and 10A is smaller than the puncture force in the case where the puncture was performed without vibrating the needle 10B. The puncture force in the case where the puncture was performed while vibrating the needle 10A is smaller than the puncture force in the case where the puncture was performed while vibrating the needle 10. In particular, the puncture force is the smallest in the case where the puncture was performed while vibrating the needle 10A in the inverse saw-tooth shape, as compared with the other cases.

As described above, it can be said that a puncture force reducing effect obtained by vibration is amplified by the grooves 15.

### (Experiment 4)

Experiment 4 was performed to confirm relation between the puncture force and a puncture velocity in each vibration in one direction (axial direction).

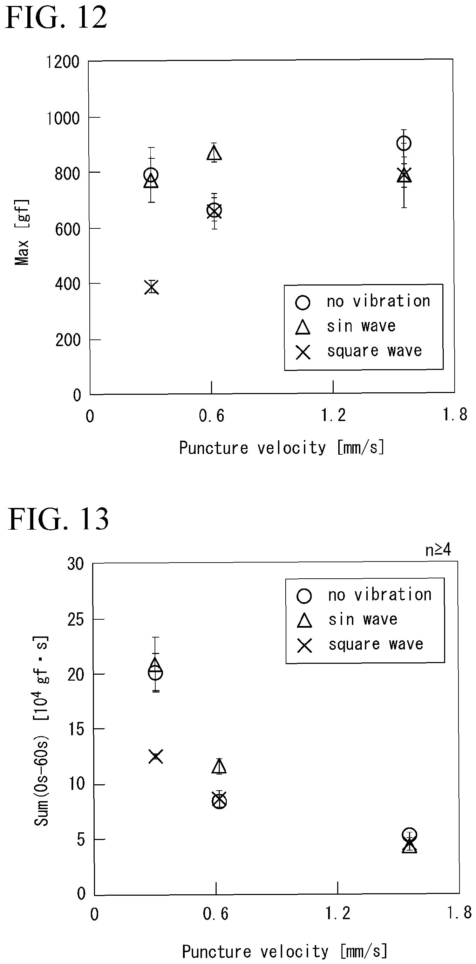

FIG. 12 is a graph showing a result of Experiment 4, which is a graph showing a correlation between the secondary peak P2 of the puncture force and a puncture velocity in each vibration.

FIG. 13 is a graph showing a result of Experiment 4, which is a graph showing a correlation between a value obtained by integrating the puncture force in each vibration from 0 s to 60 s (hereinafter, simply referred to as "total puncture force") and a puncture velocity.

Experiment 4 is different from Experiment 1 in that the kidney model 40 was punctured while vibrating the needle 10B in the axial direction. Experiment 4 was specifically performed under the following conditions. Hereinafter, descriptions of the conditions common to those of the Experiment 1 will be appropriately omitted.

In Experiment 4, only the needle 10B was used. In Experiment 4, time change of the puncture force was measured by the same method as in the Experiment 1. The puncture velocity of the needle 10B was changed to 311 pm/s, 622 pm/s, and 1556 um/s, and the time change of the puncture force was measured at each puncture velocity. When the puncture velocity was 311 um/s, the driving time of the motor 33 was set to 60s. When the puncture velocity was 622 um/s, the driving time of the motor 33 was set to 30s. When the puncture velocity was 1556 um/s, the driving time of the motor 33 was set to 15s. The secondary peak P2 of the puncture force and the total puncture force were measured at each puncture velocity, and the secondary peak P2 and the total puncture force were compared.

The Experiment 4 was performed in the case where the puncture was performed while vibrating the needle 10B in the sine wave shape in the axial direction (sin wave), and in the case where the puncture was performed while vibrating the needle 10B in the square wave shape in the axial direction (square wave).

The sine wave shape vibration has the frequency of 10 Hz and the amplitude of 100 um. The square wave shape vibration has the frequency of 10 Hz and the amplitude of 100 um.

The secondary peaks P2 of the puncture force and the total puncture force in each vibration were compared in the case where the puncture was performed without vibrating the needle 10B (no vibration).

In FIGS. 12 and 13, vertical axes represent the secondary peak P2, values of the total puncture force [gf], respectively, and horizontal axes represent the puncture velocity [mm/s].

As can be seen from the graph of FIG. 12, the secondary peak P2 of the puncture force tends to increase as the puncture velocity increases regardless of presence or absence of vibration of the needle 10B. A difference in the secondary peaks P2 of the puncture force in the case where the puncture was performed without vibrating the needle 10B and in the case where the puncture was performed while vibrating the needle 10B decreases as the puncture velocity increases.

As can be seen from the graph of FIG. 13, the total puncture force decreases in inverse proportion to the puncture velocity regardless of the presence or absence of vibration of the needle 10B. However, a difference in total puncture force in the case where the puncture was performed without vibrating the needle 10B and in the case where the puncture was performed while vibrating the needle 10B decreases as the puncture velocity increases.

As described above, it can be said that the puncture force reducing effect obtained by vibration decreases as the puncture velocity increases in the case where the needle 10B is vibrated only in one direction (axial direction).

### (Experiment 5)

Experiment 5 was performed to confirm relation between puncture force and a puncture velocity in vibration in two directions (the axial direction and the radial direction).

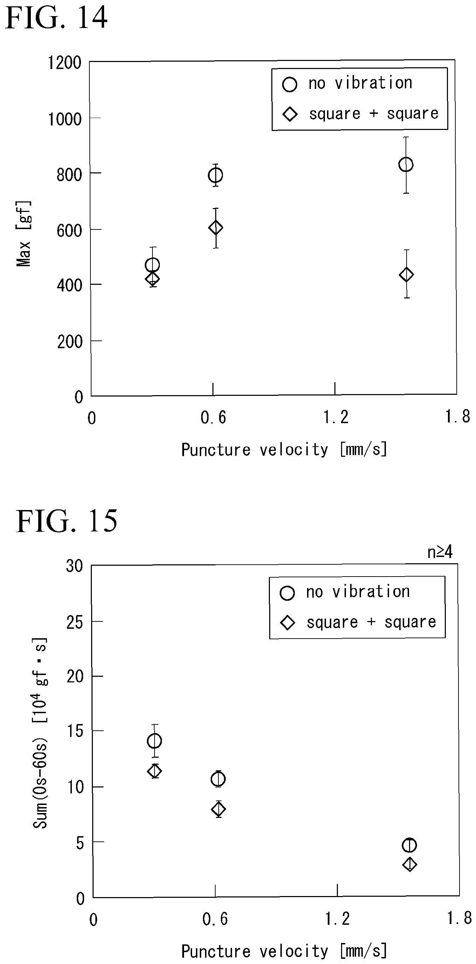

FIG. 14 is a graph showing a result of Experiment 5, which is a graph showing a correlation between the secondary peak P2 and a puncture velocity in vibration in two directions.

FIG. 15 is a graph showing a result of Experiment 5, which is a graph showing a correlation between total puncture force and a puncture velocity in vibration in two directions.

Experiment 5 was performed by the same method as in the Experiment 4. Experiment 5 is different from Experiment 4 in that the kidney model 40 was punctured while vibrating the needle 10B not only in the axial direction but also in the radial direction. Experiment 5 was specifically performed under the following conditions. Hereinafter, descriptions of the conditions common to those of the Experiment 4 will be appropriately omitted.

Experiment 5 was performed in the case where the puncture was performed while vibrating the needle 10B in the square wave shape in two directions of the axial direction and the radial direction (square + square). The vibration in the axial direction has the frequency of 10 Hz and the amplitude of 100 um. The vibration in the radial direction has the frequency of 10 Hz and an amplitude of 60 um. The vibration in the axial direction and the vibration in the radial direction are shifted from each other by a phase of π/2. Accordingly, the needle 10B vibrates by repeatedly moving and stopping in a square shape when viewed from the radial direction.

The secondary peaks P2 of the puncture force and the total puncture force in each vibration were compared with those in the case where the puncture was performed without vibrating the needle 10B (no vibration).

In FIGS. 14 and 15, vertical axes represent the secondary peak P2, values of the total puncture force [gf], respectively, and horizontal axes represent the puncture velocity [mm/s].

As can be seen from the graph of FIG. 14, in the case where the puncture was performed without vibrating the needle 10B, the secondary peak P2 of the puncture force increases as the puncture velocity increases. On the contrast, the secondary peak P2 of the puncture force in the case where the puncture was performed while vibrating the needle 10B in two directions tends to be relatively independent of the puncture velocity.

As can be seen from the graph of FIG. 15, the total puncture force decreases in inverse proportion to the puncture velocity regardless of the presence or absence of vibration of the needle 10B. At each puncture velocity, the total puncture force is reduced. A rate of reduction of the total puncture force is maintained at a constant rate without depending on the puncture velocity.

As described above, it can be said that the puncture force reducing effect obtained by vibration is constant without depending on the puncture velocity in a case where the needle 10B is vibrated in two directions (the axial direction and the radial direction).

In addition to the Experiments 1 to 5, Experiments 6 to 8 were performed.

Hereinafter, descriptions of the conditions common to those of the Experiments 1 to 5 will be appropriately omitted.

In Experiments 6 to 8, a needle (not shown) having no groove on the outer circumferential surface 14 as in the needle 10B was used. The needle of the invention is not limited to the needles 10 and 10A, and may be a needle in which no groove is formed as used in Experiments 6 to 8.

In addition to the kidney model 40, a pig kidney and a second kidney model different from the kidney model 40 were used as objects of needle puncture.

The pig kidney is a fresh one that was taken in the early morning on the day of the experiment. Therefore, the experiment was performed in a state where a membrane was formed on a surface of the pig kidney.

The second kidney model includes a spherical collagen casing and an agar gel. An outside diameter of the collagen casing is 17 mm. The inside of the collagen casing is hollow. The agar gel fills the collagen casing. A concentration of the agar gel is 2.0%. A kidney that moves in body tissue was reproduced by floating the second kidney model in a 50 mm cube container into which an aqueous solution of sodium chloride having a concentration of 3.0% by mass was poured.

The pig kidney and the second kidney model are disposed on the electronic balance 5 similarly to the kidney model 40. The pig kidney and the second kidney model disposed on the electronic balance 5 are punctured with a needle vertically downward from above. Accordingly, the puncture force of the needle, which punctures the pig kidney and the second kidney model, can be measured.

### (Experiment 6)

Experiment 6 was performed to confirm influence of vibration in the axial direction and the radial direction on the puncture force at a low frequency of 5 Hz or more and 15 Hz or less.

In Experiment 6, the pig kidney was selected as the puncture object. In Experiment 6, a needle having an outer diameter of 1.5 mm was used. A puncture velocity of the needle was 311 um/s. The needle was operated to puncture in the axial direction from a state where a tip portion of the needle was brought into contact with the puncture object. Experiment 6 was performed in the case where the puncture was performed without vibrating the needle (no vibration) and in the case where the puncture was performed while vibrating the needle in the square wave shape in two directions of the axial direction and the radial direction (biaxial square waves) . The vibration in the axial direction and the vibration in the radial direction are shifted from each other by the phase of π/2. The vibration in the axial direction has the frequency of 10 Hz and the amplitude of 100 um. The vibration in the radial direction has the frequency of 10 Hz and the amplitude of 60 um.

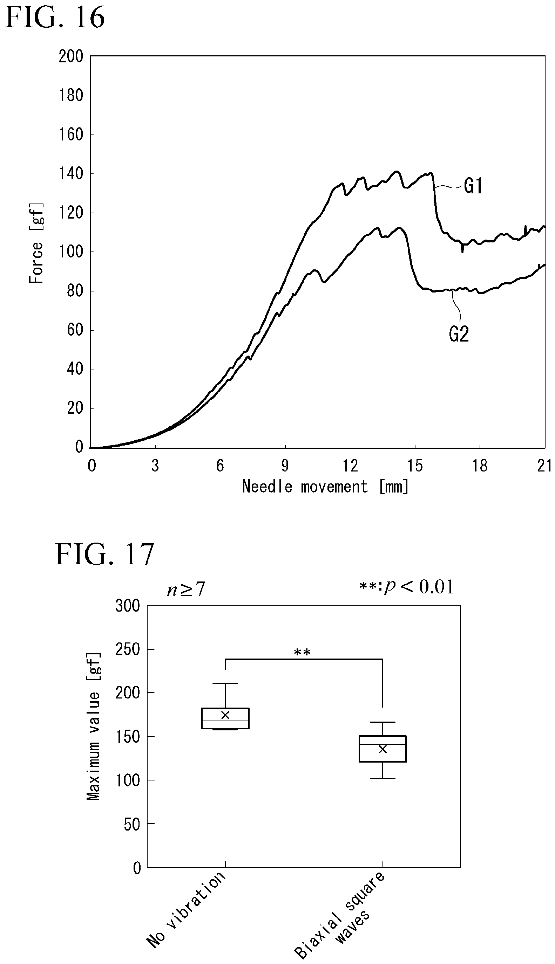

FIG. 16 is a graph showing a result of Experiment 6, which is a graph showing a correlation between puncture force and needle displacement in no vibration and in vibration in two directions. In FIG. 16, a vertical axis represents puncture force [gf], and a horizontal axis represents needle displacement [mm]. A graph G1 represents the case where the puncture was performed without vibrating the needle, and a graph G2 represents the case where the puncture was performed while vibrating the needle in the square wave shape in two directions of the axial direction and the radial direction.

As can be seen from the graph of FIG. 16, in the above two cases, as the needle displacement increased, the puncture force increased, and the needle displacement tended to be the maximum in a range of 9 mm or more and 18 mm or less. The maximum value of the puncture force is generated by elastic deformation of a membrane of the pig kidney.

In addition, it is confirmed that the puncture force can be reduced by all needle displacement in the case where the puncture was performed while vibrating the needle in the square wave shape in two directions of the axial direction and the radial direction as compared with the case where the puncture was performed without vibrating the needle.

Subsequently, Experiment 6 was tried seven times or more (sample number n ≥ 7), and a box plot diagram was created in the case where the puncture was performed without vibrating the needle and in the case where the puncture was performed while vibrating the needle in the square wave shape in two directions of the axial direction and the radial direction. The created box plot diagram is shown in FIG. 17.

FIG. 17 is a graph showing a result of Experiment 6, which is a box plot diagram comparing the maximum value of puncture force in no vibration with the maximum value of puncture force in square wave vibration. In FIG. 17, a vertical axis represents the maximum value [gf] of the puncture force, and the horizontal axis represents experimental conditions (vibration state). An upper end of a line segment passing through a box indicates the maximum value and a lower end of the line passing through the box indicates the minimum value.

Line segments of a lower side, an upper side, and between the lower side and the upper side of the box indicate a first quartile line, a third quartile line, and a second quartile line, respectively, and an x mark in the box indicates an average value.

As can be seen from FIG. 17, there was no significant difference in variation in the case where the puncture was performed while vibrating the needle in the square wave shape in two directions of the axial direction and the radial direction and in the case where the puncture was performed without vibrating the needle. On the contrast, when average values were compared, the maximum value of the puncture force was smaller in the former case than in the latter case.

By performing Student t-test, it was confirmed that a p-value is lower than a significance level even when the significance level is as small as 0.01. That is, a significant difference is observed between the average value in the former case and the average value in the latter case. Therefore, a result inevitably occurs that the puncture force was smaller in the former case than in the latter case.

A condition difference between the former case and the latter case is only the presence or absence of vibration of the needle. In consideration of the condition difference, it is considered that the puncture force can be reduced by vibrating the needle in the square wave shape in two directions of the axial direction and the radial direction as compared with the case where the puncture was performed without vibrating the needle.

### (Experiment 7)

Experiment 7 was performed to confirm influence of vibration of a needle on relation between puncture force and a puncture velocity at a frequency of 5 Hz or more and 15 Hz or less.

Experiment 7 was performed using the same needle and the same method as those in the Experiment 6. Therefore, in Experiment 7, in the case where the puncture is performed while vibrating the needle, a vibration frequency of the needle is 10 Hz. Experiment 7 is different from Experiment 6 in that measurement was performed not only when the puncture velocity of the needle was 311 um/s but also when the puncture velocity of the needle was 622 µm/s and 1556 um/s, and in that the kidney model 40 was used as a puncture object. A thickness of the silicone rubber 43 of the kidney model 40 in Experiment 7 is 600 mm or more and 700 mm or less.

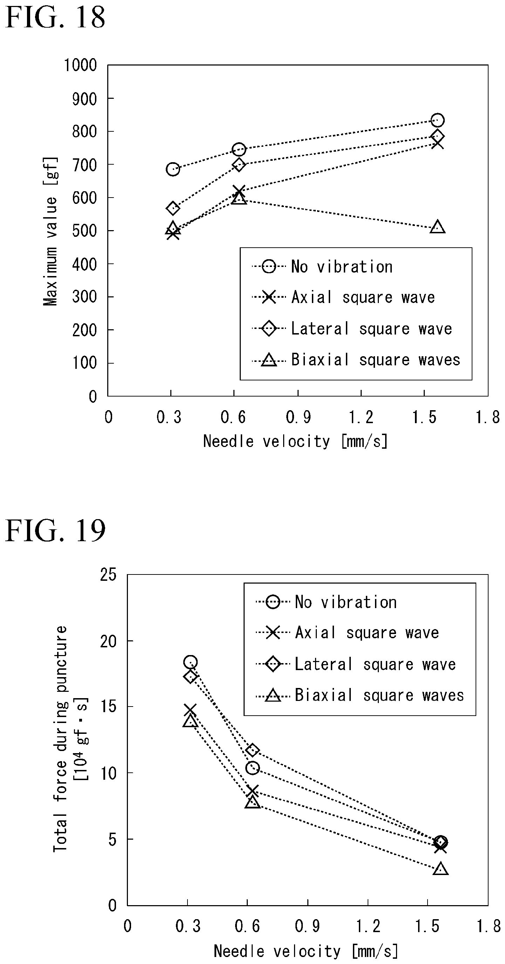

FIG. 18 is a graph showing a result of Experiment 7, which is a graph showing a correlation between the maximum value of puncture force and a puncture velocity in no vibration and in each vibration. In FIG. 18, a vertical axis represents the maximum value [gf] of the puncture force, and a horizontal axis represents the puncture velocity [mm/s]. In FIG. 18, the no vibration is denoted by "No vibration", square wave shape vibration only in the axial direction is denoted by "Axial square wave", square wave shape vibration only in the radial direction is denoted by "Lateral square wave", and square wave shape vibration in two directions of the axial direction and the radial direction is denoted by "Biaxial square waves".

As can be seen from FIG. 18, it was confirmed that in a case of vibrating the needle in at least one of the axial direction and the radial direction, the maximum value of the puncture force is smaller than in the case of no vibration at all the puncture velocities.

Accordingly, it is considered that the maximum value of the puncture force can be reduced regardless of the puncture velocity by vibrating the needle in at least one of the axial direction and the radial direction at a frequency of 5 Hz or more and 15 Hz or less.

In addition, it was confirmed that in the case of no vibration and in a case of vibrating the needle only in one of the axial direction and the radial direction, the maximum value of the puncture force increases as the puncture velocity increases. On the contrast, it was confirmed that the maximum value of the puncture force is constant in a range of about 500 gf or more and 600 gf or less at all the puncture velocities in the case of vibrating the needle in two directions of the axial direction and the radial direction.

Accordingly, it is considered that the maximum value of the puncture force can be maintained in a certain range regardless of the puncture velocity by vibrating the needle in two directions of the axial direction and the radial direction at a frequency of 5 Hz or more and 15 Hz or less.

FIG. 19 is a graph showing a result of Experiment 7, which is a graph showing a correlation between total puncture force and a puncture velocity in no vibration and in each vibration. In FIG. 19, a vertical axis represents total puncture force [10⁴ gf·s], and a horizontal axis represents a puncture velocity [mm/s]. In Experiment 7, the total puncture force is an integrated value of all puncture force applied to an object during needle puncture.

As can be seen from FIG. 19, it was confirmed that in the case of no vibration and in the case of vibrating the needle in at least one of the axial direction and the radial direction, the total puncture force is reduced as the puncture velocity increases.

In addition, it was confirmed that in the case of vibrating the needle only in one of the axial direction and the radial direction, a rate of reduction in the total puncture force decreases as the puncture velocity increases. On the contrast, it was confirmed that in a state of vibrating the needle in two directions of the axial direction and the radial direction, the total puncture force is reduced at a constant rate as the puncture velocity increases.

Accordingly, it is considered that the total puncture force can be reduced at the constant rate regardless of the puncture velocity by vibrating the needle in two directions of the axial direction and the radial direction at a frequency of 5 Hz or more and 15 Hz or less.

By Experiment 7, the puncture force reducing effect by vibrating the needle at a low frequency of 5 Hz or more and 15 Hz or less was confirmed. Further, it was confirmed that the puncture force can be further reduced and the puncture force reducing effect can be maintained constant in the case of vibrating the needle in two directions of the axial direction and the radial direction, as compared with the case of vibrating the needle only in one of the axial direction and the radial direction.

### (Experiment 8)

Experiment 8 was performed to confirm influence of acceleration of a needle on puncture accuracy.

In Experiment 8, a needle having an outer diameter of 1.5 mm and a length of 150 mm was used. The needle was operated to puncture with a stroke of 10 mm in the axial direction from a state where a tip portion of the needle was brought into contact with a puncture object. Further, the needle was accelerated in the axial direction to perform instantaneous single puncture. An acceleration in a puncture direction (axial direction) of the needle (hereinafter, simply referred to as "puncture acceleration") was changed in a range of 0 m/s² or more and about 110 m/s² or less, and displacement of the puncture object, a puncture depth, and the like were measured for each puncture acceleration. Both the second kidney model and a pig kidney were used as the puncture object.

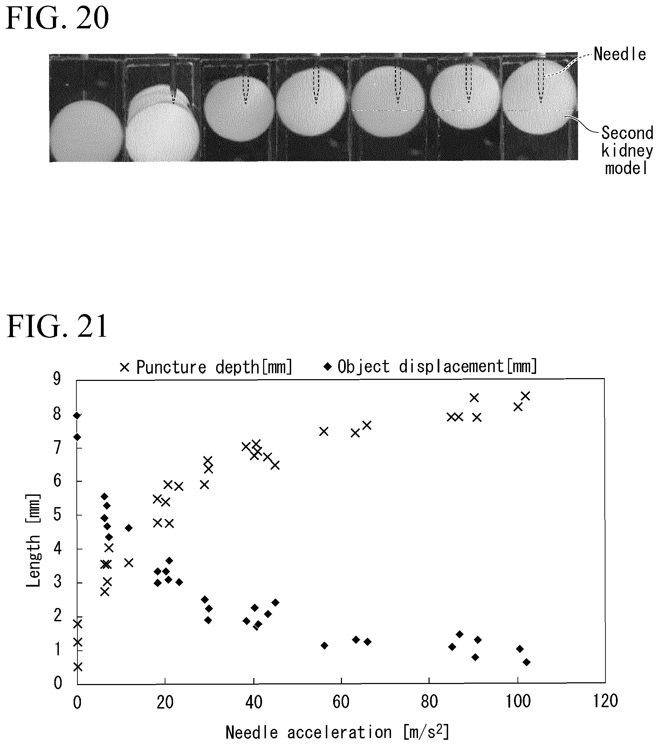

First, the second kidney model was selected as the puncture object, and the Experiment 8 was performed. A puncture velocity of the needle was set to 311 pm/s. Results of Experiment 8 using the second kidney model are shown in FIGS. 20 and 21.

FIG. 20 is a diagram showing a result of Experiment 8, which is a diagram showing displacement of the second kidney model caused by the instantaneous single puncture for each puncture acceleration. In FIG. 20, the needle and the second kidney model after puncture are displayed side by side as 0 m/s² (when the puncture is performed at a constant velocity), 6.38 m/s², 18.3 m/s², 30.0 m/s², 40.7 m/s², 65.9 m/s², and 102 m/s² in ascending order of puncture acceleration from the left. Only the second from the left in FIG. 20 (in the case where the puncture acceleration is 6.38 m/s²) shows an afterimage of the second kidney model during movement by the puncture.

As can be seen from FIG. 20, when the puncture acceleration was 0 m/s², the needle did not rupture a collagen casing, and the second kidney model sunk by an amount of the needle displacement while a tip of the needle was in contact with the collagen casing. On the contrast, when the puncture acceleration was applied to the needle, the needle ruptured the collagen casing, and the kidney model was sunk while the needle punctured the inside of the second kidney model. As the puncture acceleration increased, the displacement of the second kidney model to be punctured was reduced, and a puncture depth of the needle increased.

In order to quantitatively consider the result shown in FIG. 20, the data shown in FIG. 21 was acquired.

FIG. 21 is a graph showing a result of Experiment 8, which is a graph showing a correlation between displacement of a puncture object (denoted by Object displacement in the drawing) and a puncture acceleration in the axial direction and a correlation between a puncture depth (denoted by Puncture depth in the drawing) and the puncture acceleration in the axial direction in the second kidney model. The displacement of the puncture object means a distance of movement of the second kidney model from an initial position to the lower side due to puncture of a needle, and the puncture depth means a length by which the puncture object was actually punctured by a stroke of 10 mm. In FIG. 21, a vertical axis represents the displacement of the puncture object and the puncture depth [mm], and a horizontal axis represents the puncture acceleration [m/s²].

As can be seen from FIG. 21, in the second kidney model, as the puncture acceleration in the axial direction increased, the displacement of the puncture object decreased, and inclination of the decrease in the displacement of the puncture object gradually decreased. It was confirmed that when the puncture acceleration is 60 m/s² or more, the displacement of the puncture object is almost constant in the vicinity of 1 mm, and the puncture object hardly moves due to the puncture of the needle.

In the second kidney model, as the puncture acceleration in the axial direction increased, the puncture depth increased, and inclination of the increase in the puncture depth gradually decreased. It was confirmed that when the puncture acceleration is 80 m/s² or more, the puncture depth is almost constant in the vicinity of 8 mm, and that the puncture is performed for a length of at least 80% or more with respect to the stroke of 10 mm.

In Experiment 8 using the second kidney model, with respect to the stroke of 10 mm, a case where the puncture depth is small is considered to be low in puncture accuracy, and a case where the puncture depth is large is considered to be high in puncture accuracy. That is, the "puncture depth with respect to the stroke of 10 mm" is used as an index of the puncture accuracy. According to the index of the puncture accuracy, as the puncture acceleration increases, movement of the puncture object decreases and the puncture accuracy improves. Furthermore, when the puncture acceleration is 80 m/s² or more, the puncture accuracy is improved to such an extent that a rate of the puncture depth to the stroke is close to 100% while the movement of the puncture object is reduced to about 1 mm.

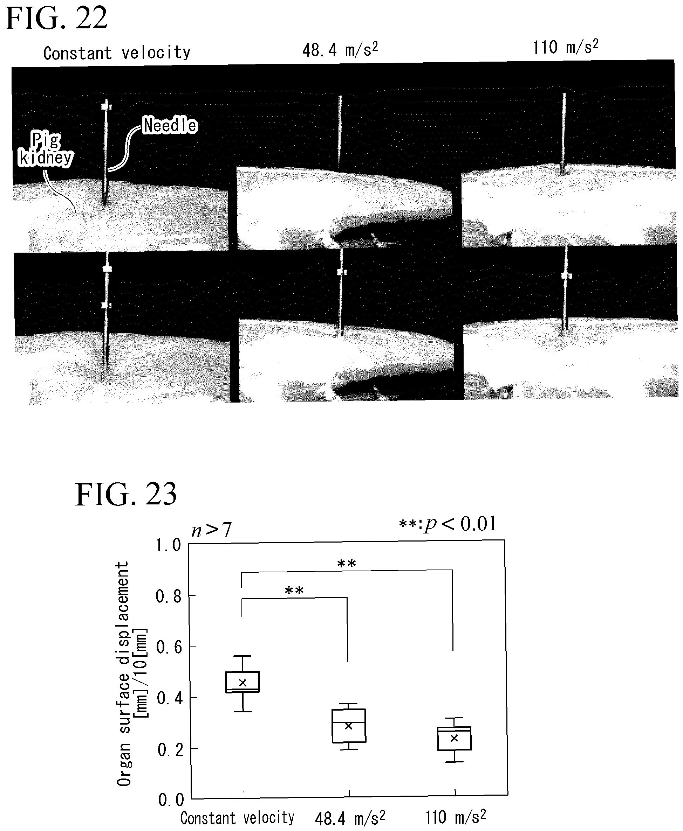

Subsequently, the pig kidney was selected as a puncture object, and the Experiment 8 was performed. A puncture velocity of the needle was changed to 3.0 mm/s. Results of Experiment 8 using the pig kidney are shown in FIGS. 22 and 23.

FIG. 22 is a diagram illustrating a result of Experiment 8, which is a diagram showing surface displacement of the pig kidney caused by instantaneous single puncture for each puncture acceleration. In FIG. 22, a needle before and after puncture and a surface of the pig kidney are displayed side by side at 0 m/s² (when the puncture is performed at a constant velocity), 48.4 m/s², and 110 m/s² in ascending order of the puncture acceleration from the left. In FIG. 22, a state before the puncture is shown in an upper part, and a state after the puncture is shown in a lower part.

As can be seen from FIG. 22, when the puncture acceleration was 0 m/s², the needle did not rupture a membrane of the pig kidney, and the membrane was deformed in a puncture direction by an amount of needle displacement while a tip of the needle was in contact with the membrane. On the contrast, when the puncture acceleration was applied to the needle, the needle ruptured the membrane of the pig kidney, and the membrane was deformed in the puncture direction while the needle punctured inside the pig kidney. As the puncture acceleration increased, the surface displacement of the pig kidney to be punctured was reduced.

In order to quantitatively consider the result shown in FIG. 22, the data shown in FIG. 23 was acquired.

FIG. 23 is a graph showing a result of Experiment 8, which is a box plot diagram showing a correlation between surface displacement of a puncture object and a puncture acceleration in the axial direction in a pig kidney. The surface displacement of the puncture object means a distance of movement of a membrane of the pig kidney from an initial position to the lower side due to puncture of a needle. FIG. 23 is a box plot diagram created by the same method as in FIG. 17 when the number n of samples is n > 7. In FIG. 23, a vertical axis represents "surface displacement of a puncture object with respect to a stroke of 10 mm" [mm]/10 [mm], and a horizontal axis represents an experimental condition (puncture acceleration).

In Experiment 8 using the pig kidney, with respect to the stroke of 10 mm, a case where the surface displacement of the puncture object is large is considered to be low in puncture accuracy, and a case where the surface displacement of the puncture object is small is considered to be high in puncture accuracy. That is, the "surface displacement of a puncture object with respect to a stroke of 10 mm" is used as an index of the puncture accuracy.

As can be seen from FIG. 23, there was no significant difference in variation in a case where the puncture was performed at a constant velocity (a case where the puncture acceleration was 0 m/s²), a case where the puncture acceleration was 48.4 m/s², and a case where the puncture acceleration was 110 m/s². On the contrast, when average values were compared, the index of the puncture accuracy decreased as the puncture acceleration increased. That is, it can be said that the puncture accuracy was improved as the puncture acceleration was increased.

Furthermore, by performing Student t-test, it was confirmed that a p-value is lower than a significance level even when the significance level is as small as 0.01. That is, a significant difference is observed between an average value in the case where the puncture was performed at the constant velocity and an average value in the case where the puncture acceleration was 48.4 m/s², and between an average value in the case where the puncture was performed at the constant velocity and an average value in the case where the puncture acceleration was 110 m/s². Therefore, the result that the puncture accuracy was improved as the puncture acceleration was increased inevitably occurs.

A condition difference in the above three cases shown in FIG. 23 is only the difference in the puncture acceleration. Considering this point, it is considered that the puncture accuracy can be improved by increasing the puncture acceleration.

According to Experiment 8, it was confirmed that the puncture accuracy can be improved by increasing the puncture acceleration in the axial direction in single puncture regardless of whether the puncture object is the second kidney model or the pig kidney. Here, as the puncture acceleration increases, the single puncture approximates to the single puncture performed by square wave vibration. The square wave vibration can be regarded as low-frequency vibration of 5 Hz or more and 15 Hz or less in the axial direction. That is, based on the results of Experiment 8, it is considered that in a case of vibrating the needle in the axial direction at a low frequency of 5 Hz or more and 15 Hz or less, the puncture accuracy can be improved by forming a vibration waveform of the needle into a square wave shape.

In Experiment 8, when the single puncture is considered to be approximate to the single puncture performed by the square wave vibration, the stroke of 10 mm is considered to be an amplitude of the square wave vibration. That is, it can be said that even with a relatively large amplitude of about 10 mm, the puncture accuracy can be improved by forming the vibration waveform of the needle into the square wave shape.

In the Experiments 6 to 8, in the case of vibrating the needle, a frequency of the vibration of the needle was set to 10 Hz, but similar results were obtained even when the frequency of the vibration of the needle was set to 5 Hz and 15 Hz.

According to the above present embodiment, the following functions and effects can be obtained.

The vibrating needle device 1 includes the vibrating unit 20 that vibrates the needle 10 along the axial direction and the radial direction.

According to this configuration, the needle 10 can be punctured into an organ in a state of causing the needle 10 to vibrate in the axial direction and the radial direction by the vibrating unit 20. The tip portion 12 of the needle 10 passes through a membrane while repeating a contact state and a non-contact state with the membrane of the organ. Accordingly, puncture resistance when the tip portion 12 passes through the membrane can be reduced as compared with a case where the needle 10 punctures the organ without vibration. Therefore, puncture force required for the tip portion 12 to pass through the membrane can be reduced. As a result, time required for the tip portion 12 to pass through the membrane after coming into contact with the membrane can be shortened.

After the needle 10 passes through the membrane of the organ, the needle 10 punctures tissue inside the organ in a vibrated state. Therefore, the needle 10 punctures the tissue while repeating a contact state and a non-contact state with the membrane. Accordingly, the membrane pressed toward the tissue by the needle 10 is restored by elastic force when the membrane is in the non-contact state with the needle 10. Therefore, the puncture resistance when the needle 10 punctures the tissue can be reduced as compared with a case where the needle 10 punctures the organ without vibration. Therefore, puncture force required for the needle 10 to puncture the tissue can be reduced.

The needle 10 punctures the tissue inside the organ while vibrating. Therefore, the needle 10 punctures the tissue while repeating a contact state and a non-contact state with the tissue. Accordingly, the tissue pressed by the needle 10 is restored by elastic force when the tissue is in the non-contact state with the needle 10. Therefore, the puncture resistance when the needle 10 punctures the tissue can be reduced as compared with the case where the needle 10 punctures the organ without vibration. Therefore, the puncture force required for the needle 10 to puncture the tissue can be reduced.

The vibrating unit 20 vibrates the needle 10 along the axial direction and the radial direction that intersects with the axial direction.

According to this configuration, it is possible to puncture the organ with the needle 10 while widening a hole opened in the organ by the needle 10 in the axial direction and the radial direction. Accordingly, the puncture force required for the needle 10 to pass through the membrane of the organ and the puncture force required for the needle 10 to puncture the tissue inside the organ can be reduced as compared with a case where the needle 10 is vibrated only in one direction.

The groove 15 is formed in the outer circumferential surface 14 of the main body portion 11 of the needle 10 over the entire circumference in the circumferential direction.

According to this configuration, a portion of the tissue pressed by the puncture of the needle 10 enters the groove 15. Accordingly, since the needle 10 is caught by the tissue, it is possible to prevent the needle 10 from being pushed out to the outside of the organ by elastic force of the tissue. Therefore, it is possible to prevent movement of the organ due to drag from the needle 10.

Since the portion of the tissue pressed by the puncture of the needle 10 enters the groove 15, the elastic force of the tissue applied to the outer circumferential surface 14 of the needle 10 can be reduced. Therefore, the puncture force required for the needle 10 to puncture the inside of the organ can be reduced.

As described above, the puncture force required for the needle 10 to puncture the membrane of the organ and the puncture force required for the needle 10 to puncture the inside of the organ can be reduced. Accordingly, the organ that is not completely fixed in the body can be prevented from moving together with the movement of the needle, and thus the needle can easily puncture the organ. Therefore, it is possible to accurately puncture a portion of an organ to be punctured, such as a lesion tissue generated in the organ.

The side surface of the groove 15 on the base end portion 13 side of the needle 10 is inclined inward in the radial direction orthogonal to the axial direction from the base end portion 13 toward the tip portion 12.

According to this configuration, when the needle 10 punctures an organ from the axial direction, the needle 10 can puncture the organ while gradually expanding the organ in the radial direction. Accordingly, the needle 10 can puncture smoothly the organ.

The vibrating unit 20 vibrates the needle 10 to have the square wave shape vibration waveform in the axial direction and the radial direction.

According to this configuration, acceleration of the needle 10 in the puncture direction (hereinafter, simply referred to as "puncture acceleration") can be increased as compared with a case where the needle 10 is vibrated with a vibration waveform such as a sine wave in which an amplitude gradually changes. Accordingly, a load applied to a membrane by the needle 10 can be increased, and thus the tip portion 12 of the needle 10 can easily pass through the membrane. Therefore, the puncture force required for the tip portion 12 to pass through the membrane can be further reduced. As a result, the time required for the tip portion 12 of the needle 10 to pass through the membrane after coming into contact with the membrane can be shortened.

In the case where the needle 10 is vibrated with the square wave shape vibration waveform, the puncture acceleration can be increased as compared with the case where the needle 10 is vibrated with a vibration waveform in which the amplitude gradually changes. Accordingly, the load applied to the membrane of the organ by the needle 10 can be increased, and thus the membrane in contact with the needle 10 can be more reliably separated from the needle 10 by the vibration of the needle 10 after the needle 10 passes through the membrane. Therefore, the membrane pressed toward tissue by the needle 10 is restored more quickly by elastic force when the membrane is in the non-contact state with the needle 10. Therefore, the puncture resistance when the needle 10 punctures the tissue can be further reduced. Accordingly, the puncture force required for the needle 10 to puncture the tissue can be further reduced. As a result, time until the membrane pressed toward the tissue by the needle 10 returns to an original position can be further shortened.

In the case where the needle 10 is vibrated with the square wave shape vibration waveform, the puncture acceleration can be increased as compared with the case where the needle 10 is vibrated with the vibration waveform in which the amplitude gradually changes. Accordingly, a load applied to the tissue inside the organ by the vibration of the needle 10 can be increased, and thus the tissue in contact with the needle 10 can be more reliably separated from the needle 10 by the vibration of the needle 10 after the needle 10 is restored. Therefore, the tissue pressed by the needle 10 can be more reliably restored by elastic force when the tissue is in the non-contact state with the needle 10. Therefore, the puncture resistance when the needle 10 punctures the tissue can be further reduced. Accordingly, the puncture force required for the needle 10 to puncture the tissue can be further reduced.

The frequency at which the vibrating unit 20 vibrates the needle 10 is set to resonate with a natural frequency of the needle 10.

According to this configuration, the frequency at which the vibrating unit 20 vibrates the needle 10 can increase an amplitude of the vibration of the needle 10 as compared with a case where the frequency is set without resonating the natural frequency of the needle 10. Accordingly, even in a situation where the vibration of the needle 10 is limited, the amplitude of the vibration of the needle 10 can be controlled by changing setting of the vibration of the vibrating unit 20. Therefore, the puncture force reducing effect of the vibrating needle device 1 can be maintained.

The first axis C1 and the second axis C2, which are axes in the vibration direction of the needle, are orthogonal to each other.

According to this configuration, the organ can be punctured in a state of vibrating the needle 10 in two orthogonal directions. Accordingly, the needle 10 can puncture while an organ is largely expanded as compared with a case where an organ is punctured in a state of vibrating the needle 10 in two intersecting directions without being orthogonal to each other. Therefore, the puncture force required for the needle 10 to pass through the membrane of the organ and the puncture force required for the needle 10 to puncture the tissue inside the organ can be reduced.

The puncture method of the needle 10 includes: a vibrating step of vibrating the needle 10 in the axial direction and the radial direction; and an insertion step of inserting the needle 10 into an organ in a state of vibrating the needle 10.

According to this configuration, the needle 10 can puncture the organ in a state of vibrating in the axial direction and the radial direction by the vibrating unit 20. The tip portion 12 of the needle 10 passes through a membrane while repeating a contact state and a non-contact state with the membrane of the organ. Accordingly, puncture resistance when the tip portion 12 passes through the membrane can be reduced as compared with a case where the needle 10 punctures the organ without vibration. Therefore, the puncture force required for the tip portion 12 to pass through the membrane can be reduced. As a result, the time required for the tip portion 12 to pass through the membrane after coming into contact with the membrane can be shortened.

After the needle 10 passes through the membrane of the organ, the needle 10 punctures tissue inside the organ in a vibrated state. Therefore, the needle 10 punctures the tissue while repeating the contact state and the non-contact state with the membrane. Accordingly, the membrane pressed toward the tissue by the needle 10 is restored by elastic force when the membrane is in the non-contact state with the needle 10. Therefore, puncture resistance when the needle 10 punctures the tissue can be reduced as compared with a case where the needle 10 punctures the organ without vibration. Therefore, the puncture force required for the needle 10 to puncture the tissue can be reduced. As a result, time until the membrane pressed toward the tissue by the needle 10 returns to an original position can be shortened.

The needle 10 punctures the tissue inside the organ while vibrating. Therefore, the needle 10 punctures the inside of the tissue while repeating the contact state and the non-contact state with the tissue. Accordingly, the tissue pressed by the needle 10 is restored by elastic force when the tissue is in the non-contact state with the needle 10. Therefore, the puncture resistance when the needle 10 punctures the tissue can be reduced as compared with the case where the needle 10 punctures the organ without vibration. Therefore, the puncture force required for the needle 10 to puncture the tissue can be reduced.

In the vibrating step, the needle 10 is vibrated along the axial direction and the radial direction that intersects with the axial direction.

According to this configuration, it is possible to puncture the organ with the needle 10 while widening a hole opened in the organ by the needle 10 in the axial direction and the radial direction. Accordingly, the puncture force required for the needle 10 to pass through the membrane of the organ and the puncture force required for the needle 10 to puncture the tissue inside the organ can be reduced as compared with a case where the needle 10 is vibrated only in one direction.

The vibrating needle device 1 includes the vibrating unit 20 that vibrates a needle at a low frequency of 5 Hz or more and 15 Hz or less along at least one of the axial direction and the radial direction.

With this configuration, it was confirmed that when the needle is vibrated at the low frequency of 5 Hz or more and 15 Hz or less along at least one of the axial direction and the radial direction, puncture force applied to a needle puncture object can be reduced.

It was confirmed that in the case of vibrating the needle at the low frequency of 5 Hz or more and 15 Hz or less, the puncture force can be further reduced by vibrating the needle along two directions of the axial direction and the radial direction as compared with the case of vibrating the needle along only one of the axial direction and the radial direction.

It was confirmed that, by vibrating the needle along two directions of the axial direction and the radial direction, the puncture force reducing effect obtained by vibration at the low frequency of 5 Hz or more and 15 Hz or less can be maintained at a constant rate regardless of a velocity.

It was confirmed that in the case of vibrating the needle at the low frequency of 5 Hz or more and 15 Hz or less, the puncture accuracy can be further improved by forming a vibration waveform of the needle into a square wave shape.

As described above, the puncture force required for the needle 10 to puncture the membrane of the organ and the puncture force required for the needle 10 to puncture the inside of the organ can be reduced. Accordingly, the organ that is not completely fixed in the body can be prevented from moving together with the movement of the needle, and thus the needle can easily puncture the organ. Therefore, it is possible to accurately puncture a portion of an organ to be punctured, such as a lesion tissue generated in the organ.

In the above embodiment, an organ such as a kidney of a human is described as an example of a needle puncture object, and the invention is not limited thereto. The organ, which is the object, may be an organ of an animal other than a human.

In the above embodiment, the vibrating needle device 1 is used to puncture an organ of a patient with the needle 10 at the time of medical care, for example, and the invention is not limited thereto. The vibrating needle device 1 may be used to assist, for example, vaccine inoculation or insulin injection of diabetes patients. As described above, the vibrating needle device 1 can reduce the puncture force and improve the puncture accuracy. Therefore, the vaccine inoculation can be easily performed even by an unskilled person. In addition, for example, when a diabetic patient performs the insulin injection by himself/herself, it is possible to insert an injection needle with less pain and with high accuracy.

In the above embodiment, the direction of the first axis C1 is coincident with the axial direction, and the direction of the second axis C2 is coincident with the radial direction, and the invention is not limited thereto. The direction of the second axis C2 may intersect the direction of the first axis C1.

In the above embodiment, the direction of the second axis C2 is orthogonal to the direction of the first axis C1, and the invention is not limited thereto. The direction of the second axis C2 may intersect the first axis C1 direction.

In the above embodiment, the maximum outer diameter of the main body portion 11 is, for example, 5 mm, and the invention is not limited thereto. The maximum outer diameter of the main body portion 11 may be 2 mm or more and less than 5 mm.

In the above embodiment, the vibrating unit 20 includes the piezoelectric actuator 22, and the invention is not limited thereto. The vibrating unit 20 may be any unit as long as it vibrates the needle 10B, and may be, for example, a leaf spring.

In the above embodiment, the vibrating unit 20 vibrates the needle 10 to have the square wave shape vibration waveform in the axial direction and the radial direction, and the invention is not limited thereto. For example, the vibrating unit 20 may vibrate the needle 10 to have the square wave shape vibration waveform in at least one of the axial direction and the radial direction.

For example, the vibrating unit 20 may vibrate the needle 10 to have a saw-tooth shape or/and inverse saw-tooth shape vibration waveform in at least one of the axial direction and the radial direction.

According to this configuration, acceleration of the needle 10 in the puncture direction can be increased as compared with the case where the needle 10 is vibrated with a vibration waveform such as a sine wave in which an amplitude gradually changes. Accordingly, a load applied to a membrane by the vibration of the needle 10 can be increased, and thus the tip portion 12 of the needle 10 can easily pass through the membrane. Therefore, the puncture force required for the tip portion 12 to pass through the membrane can be further reduced. As a result, the time required for the tip portion 12 of the needle 10 to pass through the membrane after coming into contact with the membrane can be shortened.

In the case where the needle 10 is vibrated with the saw-tooth shape or/and inverse saw-tooth shape vibration waveform, the puncture acceleration can be increased as compared with the case where the needle 10 is vibrated with the vibration waveform in which the amplitude gradually changes. Accordingly, the load applied to the membrane of the organ by the needle 10 can be increased, and thus the membrane in contact with the needle 10 can be more reliably separated from the needle 10 by the vibration of the needle 10 after the needle 10 passes through the membrane. Therefore, the membrane pressed toward tissue by the needle 10 is restored more quickly by elastic force when the membrane is in the non-contact state with the needle 10. Therefore, the puncture resistance when the needle 10 punctures the tissue can be further reduced. Accordingly, the puncture force required for the needle 10 to puncture the tissue can be further reduced. As a result, time until the membrane pressed toward the tissue by the needle 10 returns to an original position can be further shortened.

In the case where the needle 10 is vibrated with the saw-tooth shape or/and inverse saw-tooth shape vibration waveform, the puncture acceleration can be increased as compared with the case where the needle 10 is vibrated with the vibration waveform in which the amplitude gradually changes. Accordingly, a load applied to the tissue inside the organ by the vibration of the needle 10 can be increased, and thus the tissue in contact with the needle 10 can be more reliably separated from the needle 10 by the vibration of the needle 10 after the needle 10 is restored. Therefore, the tissue pressed by the needle 10 can be more reliably restored by elastic force when the tissue is in the non-contact state with the needle 10. Therefore, the puncture resistance when the needle 10 punctures the tissue can be further reduced. Accordingly, the puncture force required for the needle 10 to puncture the tissue can be further reduced.

In addition, it is possible to appropriately replace components in the above embodiments with known components without departing from the spirit of the invention, and the embodiments mentioned above may be suitably combined.

### Industrial Applicability

The invention relates to a vibrating needle device and a needle insertion method. According to the invention, it is possible to provide the vibrating needle device and the needle insertion method that can easily puncture an object with a needle.

### Reference Sign List

1: vibrating needle device
10: needle
11: main body portion
12: tip portion
13: base end portion
14: outer circumferential surface
15: groove
20: vibrating unit
C1: first axis
C2: second axis

## Claims

1. A vibrating needle device comprising:
a needle including a main body portion extending in a direction of a first axis and having a groove formed in an outer circumferential surface over an entire circumference in a circumferential direction around the first axis and a tip portion provided at one end of the main body portion and tapered in a direction away from the main body portion in the direction of the first axis; and
a vibrating unit configured to vibrate the needle along the direction of the first axis and a direction of a second axis that intersects with the direction of the first axis.

2. The vibrating needle device according to claim 1, wherein
a side surface of the groove on a base end portion side of the needle is inclined inward in a radial direction orthogonal to the first axis from the base end portion side toward a tip portion side.

3. A vibrating needle device comprising:
a needle including a main body portion extending in a direction of a first axis and a tip portion provided at one end of the main body portion and tapered in a direction away from the main body portion in the direction of the first axis; and
a vibrating unit configured to vibrate the needle at a low frequency of 5 Hz or more and 15 Hz or less along at least one of the direction of the first axis and a direction of a second axis that intersects with the direction of the first axis.

4. The vibrating needle device according to any one of claims 1 to 3, wherein
the vibrating unit is configured to vibrate the needle to have a saw-tooth shape or/and inverse saw-tooth shape vibration waveform in at least one of the direction of the first axis and the direction of the second axis.

5. The vibrating needle device according to any one of claims 1 to 4, wherein
the vibrating unit is configured to vibrate the needle to have a square wave shape vibration waveform in at least one of the direction of the first axis and the direction of the second axis.

6. The vibrating needle device according to any one of claims 1 to 5, wherein
a frequency at which the vibrating unit vibrates the needle is set to resonate with a natural frequency of the needle.

7. The vibrating needle device according to any one of claims 1 to 6, wherein
the second axis is orthogonal to the first axis.

8. A needle insertion method using the vibrating needle device according to any one of claims 1 to 7, the needle insertion method comprising:
a vibrating step of vibrating the needle in the direction of the first axis and the direction of the second axis; and
an insertion step of inserting the needle into an object in a state of vibrating the needle.

9. A needle insertion method using the vibrating needle device according to claim 3, the needle insertion method comprising:
a vibrating step of vibrating the needle at a low frequency of 5 Hz or more and 15 Hz or less in at least one of the direction of the first axis and the direction of the second axis; and
an insertion step of inserting the needle into an object in a state of vibrating the needle.
